# EUROPEAN PATENT SPECIFICATION

(11) **EP 1 343 815 B1**
(45) Date of publication and mention of the grant of the patent: **18.03.2009**
(21) Application number: 01272120.5
(22) Date of filing: 24.12.2001
(51) Int. Cl.: C07K 14/44

(54) **ANTIBACTERIAL TREATMENTS**
ANTIBAKTERIELLE BEHANDLUNGSMETHODEN
TRAITEMENTS ANTIBACTERIENS

(30) Priority: 22.12.2000 GB 0031425
(43) Date of publication of application: 17.09.2003
(73) Proprietor: Amoebics Limited, Ashkirk, Selkirkshire TD7 4NY (GB)
(72) Inventor: MACIVER, Sutherland, Kester, Penicuik EH26 9JB (GB)
(74) Representative: Szczuka, Jan Tymoteusz
(86) International application number: PCT/GB2001/005783
(87) International publication number: WO 2002/051866

(56) References cited:
- WO-A-97/18826
- US-A- 5 942 110
- MAHANA WAHIB: "Mapping of staphylococcal enterotoxin A functional binding sites and presentation by monoclonal antibodies and fusion proteins." INFECTION AND IMMUNITY, vol. 67, no. 4, April 1999 (1999-04), pages 1894-1900, XP002206047 ISSN: 0019-9567
- IGARASHI MASAYUKI ET AL: "Vinylamycin, a new depsipeptide antibiotic, from Streptomyces sp." JOURNAL OF ANTIBIOTICS (TOKYO), vol. 52, no. 10, October 1999 (1999-10), pages 873-879, XP001085499 ISSN: 0021-8820
- DROZANSKI W: "BACTERIOLYTIC ENZYME PRODUCED BY ACANTHAMOEBA-CASTELLANII" ACTA MICROBIOLOGICA POLONICA SERIES A MICROBIOLOGIA GENERALIS, vol. 1, no. 3-4, 1969, pages 155-168, XP001084986 ISSN: 0567-7815 cited in the application
- SUN W.; BROVKO L.; GRIFFITHS M.: 'USE OF BIOLUMINESCENT SALMONELLA FOR ASSESSING THE EFFICIENCY OF CONSTRUCTED PHAGE-BASED BIOSORBENT' JOURNAL OF INDUSTRIAL MICROBIOLOGY AND BIOTECHNOLOGY, BASINGSTOKE, GB vol. 25, November 2000, pages 273 - 275
- BODER ERIC T.; MIDELFORT K.S.; WITTRUP K.D.: 'Directed evolution of antibody fragments with monovalent femtomolar antigen-binding affinity' PROCEEDINGS OF THE NATIONAL ACADEMY OF SCIENCES OF USA vol. 97, no. 20, 26 September 2000, pages 10701 - 10705
- VENKATARAMAN CHANDRASEKAR ET AL: 'Identification of a Gal/GalNAc lectin in the protozoan Hartmannella vermiformis as a potential receptor for attachment and invasion by the Legionnaires' disease bacterium' JOURNAL OF EXPERIMENTAL MEDICINE vol. 186, no. 4, 1997, pages 537 - 547
- KENDREW: 'The encyclopedia of molecular biology', BLACKWELL SCIENCE * page 527 - page 528 *
- KWAIK ET AL: 'Signal transduction in the protozoan host Hartmanella vermiformis upon attachment and invasion by Legionella micdadei' APPL. ENV. MICROBIOL. vol. 64, no. 9, 1998, pages 3134 - 3139

## Description

The present invention relates to novel antibacterial agents and products and their use in combating bacteria, including in diagnostic systems.

In recent years many bacteria have become increasingly resistant to antibiotics. One such bacterium which is particularly hazardous is Staphylococcus aureus (SA), especially the strains commonly referred to as MRSA (methicillin-resistant Staphylococcus aureus) which is increasingly commonly found in hospital environments. S. aureus is a particularly dangerous pathogen on account of its ability to "hide" from the body's immune system.

In more detail, the body's immune system normally makes antibodies to invading bacteria which bind onto the bacteria at their Fab ends and the body's white blood cells are then able to phagocytose the bacteria by binding to the other (Fc) ends of the antibodies. Recognition of the SA bacterium is a problem for the immune system as the bacterium is able to cover itself in the host's own antibodies, by binding onto them by the Fc part(i.e. the wrong way found) ! The bacteria thus avoid being phagocytosed by the white blood cells that cannot now bind onto the Fc ends of the antibodies. This difficulty is compounded by the fact that the surface of the bacterium is substantially non-allergenic so that the immune system cannot make good antibodies against it in the first place.

Sun et al (J. Indust. Microb. & Biotech. (200), 25, p173-175) describe a bacteriophage-based biosorbent for *Salmonelk* enteritidis for capturing said bacteria for the purposes of detecting and confirming the presence of pathogens in food.

Kwaik et al (Applied & Environmental Microbiology (1998) 64(a) p3134-3139 discloses a method whereby there is a non-specific binding of a bacteria to an amoebae and then subsequent lysing of the amabae in order that the components of the lysate can be studied.

Venkataraman et al (J. Experiment. Med. (1997) 186(4), p537 - 547) describes the isolation of a receptor by immunoprecipitation after lysing a protozoa, using an antibody to a particular lectin in order to form an immuno complex which may be isolated.

It is an object of the present invention to avoid or minimize one or more of the above problems or disadvantages.

Constructed Phage-based Biosorbent'. In more detail, Sun et al discloses the use of a bacteriophage which can specifically bind Salmonella bacteria, to capture such bacteria from food samples, for the purposes of detecting and confirming the presence of pathogens in food. There is no suggestion of any of the steps of lysing the bacteriophage, of separating any complex from any lysate, of treating any complex so as to release any bacterium binding component, or of recovering any bacterium binding component or peptide from any eukaryotic micro-organism for any purpose at all, let alone for any therapeutic purpose.

It is an object of the present invention to avoid or minimize one or more of the above problems or disadvantages.

We have now found that certain eukaryotic micro-organisms, and especially micro-organisms of the type which are predators of bacteria, have receptor components which can bind such bacteria with high specificity and high affinity, in contrast to the relatively non-specific binding which is frequently to be found between eukaryotic micro-organisms and other biomolecules or other materials. We have further found that such bacterial binding components from certain eukaryotic bacterium predator micro-organisms can be identified and isolated and used as a basis for a new type of antibacterial agent antibiotic that works together with the body's own immune system. In more detail we use these components to bind onto the bacteria to act as a flag, drawing the bacteria to the attention of passing white blood cells that can then destroy them by phagocytosis in the normal way.

In one aspect the present invention provides a method of recovering a bacterium binding component suitable for use in recovering said at least one bacterium binding component of said eukaryotic micro-organism.

Preferably the present invention provides a method of providing a bacterium binding component suitable for use in the preparation of a product for use in combating a target bacterium (including the inactivation of said bacterium and/or the treatment or diagnosis of an infection by said bacterium), which method comprises the steps of:
providing an eukaryotic micro-organism candidate for binding of said target bacterium;
contacting bacterium binding components of said eukaryotic micro-organism with said target bacterium for binding of said target bacterium with at least one said bacterium binding component, and
lysing said eukaryotic micro-organism;
separating the bacterium with any said eukaryotic micro-organism bacterium binding components bound thereto, from the remainder of said lysate;
treating the separated out bacterium so as to release said at least one bacterium binding component of said eukaryotic micro-organism from said bacterium; and
recovering said at least one bacterium binding component of said eukaryotic micro-organism.

Suitable candidate predator eukaryotic micro-organisms are more or less widely found in diverse soil and other habitats of bacteria. In a preferred aspect the method of the present invention includes the preliminary step of recovering at least one eukaryotic micro-organism from a habitat of said bacterium. In general the method of the present invention also includes the further preliminary step of screening a plurality of eukaryotic micro-organisms for at least one eukaryotic micro-organism binding said target bacterium. Conveniently said screening comprises culturing said target bacterium in contact with said eukaryotic micro-organisms in the substantial absence of nutrient medium and selecting eukaryotic micro-organisms which thrive.

The amoebae which we have used in the screen to detect and identify suitable bacterial binding proteins were from a large collection (i.e. 400) of cloned cultures obtained from soil samples from all over the world. The strain of Acanthamoeba ("Ven") used in Example 1 hereinbelow and which was used to obtain the S.aureus bacterial binding peptide with the amino acid sequence disclosed hereinbelow, was isolated from soil from Venezuela in 1996. We have, though, also identified Acanthamoebal strains from soils from Bilston, Midlothian, Scotland; Bearsden, Glasgow, Scotland and Glastonbury, England all of which had similar properties with respect to their ability to bind and phagocytose S. aureus. The "Ven" strain was chosen from this short list of otherwise suitable candidates because of its superior ability to grow and be maintained in axenic culture conditions.

Similarly, our screen for Entercoccus consuming amoeba produced a large number of strains. There were many more amoeba that consumed this bacteria compared to S. aureus and so it was necessary to screen a very limited sub-set of the full library of amoebae strains. Examples of strains of Acanthamoeba that were found to be suitable are, Port Louis, Mauritius; Bodrum, Turkey; Eddleston, Scotland; Peebles, Scotland. The strain from Mauritius was ultimately chosen again because of its ability to thrive in axenic medium.

The eukaryotic micro-organism candidates may be contacted with the target bacterium binding components either before or after lysis of the eukaryotic micro-organisms i.e. while the bacterium binding components are present on an intact eukaryotic micro-organisms, or while they are present in the form of larger or smaller fragments of a lysate of said eukaryotic micro-organisms. Advantageously the target bacteria are fixed prior to use in the method of the present invention (as a preliminary step) so as make them resistant to lysis during lysis of the eukaryotic micro-organisms where this is carried out after contacting of the target bacteria with the eukaryotic micro-organism bacterial binding components, and to make them resistant to lysis by the release agents used to separate said at least one bacterium binding component of said eukaryotic micro-organism from said bacterium. The latter has the advantage of substantially simplifying the task of recovery of the released bacterium binding component of said eukaryotic micro-organism as it is much simpler to separate bacterium binding components from whole bacterium than from a bacterial lysate.

It is a particular advantage of the present invention that it can provide bacterium binding components with high binding specificity and affinity. In a preferred form of the method of the present invention, therefore, there is included the further step of treating the separated out bacterium so as to release differentially any material bound thereto with low specificity and affinity, prior to release of said bacterial binding components with high binding specificity and affinity. In general we have found that the preferred bacterium binding components with high binding specificity and affinity require the use of high ionic strength buffers with strong detergents. One particularly suitable stripping or release buffer well known in the art comprises 1% Sodium Deoxycholate, 0.1% Sodium dodecyl sulphate, 1% Triton, 10mM Tris pH8.0, 0.14M NaCl and 1mM NaN₃ and is generally known in the art as RIPA buffer, Various different release buffers could be used for the initial removal of low specificity and affinity bound material, such as for example low ionic strength buffers such as 20mM Tris pH8.0, or relatively weak detergents such as 0.1% NP40 in 20mM Tris pH8.0.

In general the bacterial binding components of the present invention are substantially proteinaceous in nature i.e. comprise polypeptide chains of various different lengths, with possibly other moieties such as sugars attached thereto.

Various different terms are used in the art such as peptides, oligopeptides, polypeptides and proteins to suggest differences in polypeptide length but there are no precise definitions of these terms. For the purposes of convenience we have generally used the term "peptide" herein to indicate polypeptide molecules of any and all chain lengths, unless the context specifically requires otherwise. For the avoidance of doubt therefore, the use of the term "peptide" or "protein" should not be interpreted as indicating any particular chain length or range of chain length unless the context specifically requires otherwise. Thus the bacterial binding components according to the present invention generally comprise bacterial binding peptides which may conveniently be referred to herein as BBPs.

It will be appreciated that bacterium binding components obtained in the abovedescribed method of the invention may include inactive regions not specifically involved in binding to the bacterium. Preferably therefore, the (initially) recovered bacterium binding component is further broken down and screened again against said bacterium for identification of active bacterium binding component regions. It should be noted though that the relatively high binding specificity and affinity achievable with the present invention does generally depend to a greater or lesser degree on the conformation of the peptide chain of the bacterial binding component. Thus whilst some degree of binding may still be achievable with relatively short peptide chains, it is generally preferred that there should be used a peptide chain having a length not less than that required to provide a stable folding unit under normal physiological conditions. Typically this could be a chain length not less than 80 amino acids, and often than 100 amino acids. Nevertheless, in some cases a BBP with high specificity and high affinity could be constituted by a considerably shorter polypeptide chain. Desirably also the bacterial binding peptides or proteins should not have any extraneous chain portions, especially any which protrude from the stable folding unit involved in bacterial binding, in order to minimise the antigenicity of the bacterial binding peptides or proteins and avoid unnecessarily provoking the immune system of the patient undergoing treatment.

In accordance with the present invention it will be appreciated that whilst it is relatively easy in practice to identify micro-organisms which are predators for target bacteria, it is also possible that some micro-organisms which have such high-specificity and high affinity binding for target bacteria, may not for some reason be able to thrive by using such bacteria as a (sole) source of nutrient or, may not ingest the bacteria following binding. Such micro-organisms may nevertheless also be identified without undue difficulty by suitable techniques such as co-sedimentation of the bacteria with the amoebae, as further described hereinbelow, and accordingly the use of such micro-organisms as a source of bacterial binding components is also encompassed by the present invention.

Thus by means of the present invention it is possible to obtain bacterial binding peptides which can be used in various different ways to combat bacteria, including those resistant to existing anti-biotic treatments and the like. It will be appreciated that BBPs binding different bacteria may be obtained from different micro-organism predators. Nevertheless it is also possible to obtain specific individual BBPs capable of binding to various different bacteria. In this connection we have found that a BBP obtained from the Ven Strain of Acanthamoeba with an active bacterial binding domain having the following amino acid sequence was found to bind seven different strains of Staphylococcus aureus.

GSTGVHLDDVVIGSFQASPRQVSVSLSCFGDSGKPSGPMVHHVAGSELMAFSRIAFESASSQ
SHYLGAGFQRLRASGACPWGHGAWPCGPYLHPEGHCPGQVQHRMPVKAGVRLVDCPGRTGVV
VGHRVPQVCPVQSIIGIAVPRTGRRHVVREWTMNIA

In addition this particular BBP was also found to bind efficiently other bacteria. The same BBP was further found to have limited binding with Pseudomonas fluorescens, Bacillus subtilis and Escherichia coli however, a similarly sized BBP has been obtained from another micro organism predator, viz Acanthamoeba palestinensis which does yield a BBP with good binding efficiency for Pseudomonas fluorescens. Yet another BBP has been obtained from an Acanthamoeba strain which binds Enterococcus faecialis. In practice, eukaryotic micro-organism predators such as amoebae are available for many different bacterial targets, and the present invention also provides BBPs with high specificity and high affinity binding for a very wide range of target bacteria, including inter alia Enterococcus and Streptococcus bacteria, as well as other bacteria of interest in medicine such as E. coli 0157. In general target bacteria of particular interest are those which are pathogenic to man and/or domesticated animals, and especially those difficult to phagocytose or otherwise neutralise by the infected subject's immune system.

It will also be appreciated that while it is generally most convenient (for reasons of inter alia ease of separation from unbound material etc.) to use whole bacteria for binding with eukaryotic micro-organism bacterium binding components in the method of the invention, it will be appreciated that it would, in principle, also be possible to use isolated bacterial cell walls, or even fragments of bacterial cell walls. Methods of isolating bacterial cell walls, and of breaking bacterial cell walls up into their constituents, are well known in the art.

It is believed that the high binding specificity and affinity of the bacterial binding components of the present invention is due to their substantially proteinaceous nature. Nevertheless these components may also include non-proteinaceous moieties such as glycosidic moieties which are found in glycosylated proteins, and accordingly references to bacterial binging peptides or proteins herein are intended to encompass peptides or proteins having such moieties bonded thereto.

The production and use of derivatives, analogues, and peptides related to the bacterium binding component provided by the present invention are also envisioned and are within the scope of the present invention. Such derivatives, analogues, and peptides which exhibit bacteria binding activity are also useful in combating bacteria. Such derivatives, analogues, or peptides may have increased or reduced biological activities in comparison to native bacteria binding components. Such derivatives, analogues, and peptides of the present invention can be produced by a variety of means known in the art.

Various methods for recovering bacterium binding components in the above described method of the invention can be used and include those commonly used in biochemistry such as one or more of centrifugation, chromatography, and polyacrylamide gel electrophoresis (PAGE). The chromatography methods used can include, but are not limited to, combinations of ion exchange, gel permeation, and affinity chromatography based on hydrophobicity, immunoaffinity or other affinity interactions. All of the chromatography methods can include both low pressure and high pressure techniques.

The bacterium binding components of the present invention can now also be produced by recombinant DNA techniques or chemical synthetic methods. To produce them by recombinant methods, messenger RNA (mRNA) for the preparation of complementary DNA (cDNA) can be obtained from the micro-organisms that produce the bacteria binding components. Either cDNA or genomic libraries can be prepared from DNA fragments generated using techniques well known in the art and/or are readily available commercially. The fragments which encode the bacteria binding components can be identified by screening the libraries with a nucleotide probe which would encode an amino acid sequence homologous to the amino acid sequence of a bacteria binding component, or active region thereof, provided by the present invention, e.g. an amino acid sequence such as that shown in hereinbefore. Although portions of the coding sequence may be utilized for cloning and expression, full length clones, may be preferable for expression. Techniques well known to those skilled in the art may be used for the isolation of DNA, generation of appropriate fragments, by various methods, construction of clones and libraries, and screening recombinants can be used. See, for example, the techniques described in "Molecular Cloning. A Laboratory Manual" Authors, Sambrook, Fritch & Maniatis. Cold Spring Harbour Laboratory Press. 1989.

Due to the degeneracy of the nucleotide coding sequences, alternative DNA sequences which encode analogous amino acid sequences for a bacteria binding component gene can be used in the practice of the present invention for the cloning and expression of bacteria binding components. Such alterations include deletions, additions or substitutions of different nucleotide residues resulting in a sequence that encodes the same or a functionally equivalent gene product. The gene product may contain deletions, additions or substitutions of amino acid residues within the sequence, which result in a silent change thus producing a bioactive product. Bioactivity in this context is measured by the ability of the gene product to bind a target bacterium.

Any amino acid substitutions in the bacterium binding components can be made on the basis of similarity in polarity, charge, solubility, hydrophobicity/hydrophilicity, size, conformation etc. of the residue involved. For example, negatively charged amino acids include aspartic and glutamic acid; positively charged amino acids include lysine and arginine; amino acids with uncharged polar head groups having similar hydrophilicity values include the following: leucine, isoleucine, valine; glycine, alanine; asparagine, glutamine; serine, threonine; phenylalanine, tyrosine.

In order to express a biologically active bacterium binding component, the nucleotide sequence encoding it, or a functionally equivalent nucleotide sequence, is inserted into an appropriate vector, i.e., a vector which contains the necessary elements for the transcription and translation of the inserted coding sequence. Modified versions of the sequence can be engineered to enhance stability, production, purification, yield or toxicity of the expressed product.

Methods which are well known to those skilled in the art can be used to construct expression vectors containing a bacteria binding component coding sequence and appropriate transcriptional/translational control signals. These methods include in vitro recombinant DNA techniques, synthetic techniques and in vivo recombination/genetic techniques. See, for example, the techniques described in "Molecular Cloning. A Laboratory Manual" Authors, Sambrook, Fritch & Maniatis. Cold Spring Harbour Laboratory Press. 1989.

A variety of host-expression systems can be utilized to express the bacteria binding component coding sequence. These include, but are not limited to, micro-organisms, such as bacteria (e.g. Escherichia coli) transformed with a recombinant bacteriophage DNA, plasmid DNA or cosmid DNA expression vector containing the bacteria binding component coding sequence; yeast (e.g. Pichia pastoris) transformed with recombinant yeast expression vectors containing the coding sequence; plant, animal and insect cell systems infected with recombinant virus expression vectors or transformed with recombinant plasmid expression vectors, such as Ti plasmid, containing the coding sequence.

Preferably a yeast-expression system is utilised because it is advantageous in being free of containing lipopolysaccharide (LPS). LPS is a bacterial component which acts as a polyclonal mitogen and is pyrogenic in very small quantities. Where a bacterial-expression system is utilised it is desirable to purify the product of the bacterial LPS prior to subsequent therapeutic use so as to minimise potential adverse immune responses. Where a yeast-expression system is used this has the advantage that the protein product may be simply and reliably purified from the culture medium by using more or less straightforward procedures, such as precipitation, dialysis, chromatography and gel filtration.

Eukaryotic gene expression systems such as yeast are further preferable because resulting protein products are frequently produced in a glycosylated form which increases the likelihood of providing a protein product with a substantially full native biological function.

In addition, a yeast system provides high yields and the ease of purification of the desired product results in economies of production compared with systems such as bacterial-expression systems. Advantageously, the expression construct in a yeast system integrates into the yeast host genome providing a resource of substantially genetically stable host. This is particularly important where the expression system is performed on an industrial scale.

Depending on the host/vector system utilized, any of a number of suitable transcription and translation elements including constitutive and inducible promoters, transcription enhancer elements, transcription terminators, etc., can be used in the expression vector.

In addition to producing bacteria binding components by recombinant DNA techniques, they can also be produced in whole or in part by solid phase chemical synthetic techniques based on the determined amino acid sequence (e.g. using the Fmoc/tBu system). This approach may be particularly useful in generating segments or fragments of a bacterium binding component corresponding to one or more of its biologically active regions.

Advantageously the above described method of the invention also includes the further steps of: sequencing a recovered bacterium binding component peptide, so as to obtain the amino acid sequence thereof;
obtaining DNA (e.g. cDNA) coding for said bacterium binding component amino acid sequence;
introducing said DNA into an expression vector; and recovering recombinant bacterium binding component peptide produced by said expression vector.

The present invention also provides a cDNA sequence encoding the active bacterium binding domain of the protein isolated from the amoeba and having the above described amino acid, which cDNA has the following nucleotide sequence.

GGCTCCACGGGAGTCCATCTGGACGACGTCGTCATCGGCAGTTTCCAGGCCAGCCCTCGTCA
GGTAAGTGTTAGCCTGAGTTGCTTTGGAGACTCAGGAAAACCTAGTGGGCCCATGGTGCACC
ATGTTGCAGGCTCAGAGTTGATGGCCTTCTCCCGGATCGCGTTCGAATCAGCCTCGAGCCAG
TCGCACTACCTGGGTGCAGGATTCCAGAGGTTGAGAGCTTCCGGAGCTTGCCCTTGGGGGCA
TGGTGCTTGGCCCTGTGGTCCCTACCTGCACCCAGAGGGCCATTGCCCGGGACAGGTCCAGC
ATCGGATGCCCGTCAAGGCGGGTGTCAGACTCGTCGACTGCCCGGCCGGACTGGCGTCGTT
GTGGGCCATCGGGTTCCACAGGTGTGTCCGGTTCAGTCAATCATAGGCATTGCTGTTCCAAG
GACAGGACGCCGCCATGTTGTTCGGGAGTGGACCATGAACATCGCC

In general a predator for a given target bacterium can be identified by introducing the candidate predator micro-organism(s) to the target bacterium, and selecting the predator micro-organisms which thrive. As noted above, certain eukaryotic micro-organisms which have suitable bacterial binding components, may nevertheless be unable to consume the bacteria and thrive on them. Such micro-organisms may nevertheless be identified by use of relatively straightforward techniques such as differential centrifugation. Thus, typically the amoeba or other eukaryotic bacteria-binding micro-organism would be contacted with the target bacteria, and then subjected to a differential centrifugation using for example a suitable density gradient and/or centrifugation speed such as to selectively sediment out the eukaryotic micro-organism and not any free unbound bacteria. The sedimented out pellet can then be simply tested for the presence of any bacteria which would,only be found if they had been bound by the micro-organism. Suitable procedures for testing for the presence of bacteria are well known in the art such as quantitation by dilution followed by culturing on plates, or by the use of strains. Binding specificity and capacity can be measured by a comparison of the numbers of bacteria that are found to be in the pellet (bound to the micro-organism) compared to the numbers in the supernatant.

Suitable eukaryotic predator (and other bacterium binding) micro-organisms generally include amoeba, and protozoa such as ciliates and flagellates, and may be recovered from typical bacteria habitats such as soil, especially soil relatively rich in organic matter. Particular types of soil habitat are generally preferred,for particular types of predator micro-organism. In the present case we have found it particularly convenient to use amoebae that can be cultured axenically in liquid medium. Such amoebae generally favour habitats which are subjected to alternating wet and dry periods.

There is a large number of different amoebal genera which have suitable binding components. The Acanthamoeba and Naegleria genera are however particularly convenient due to their relatively great abundance, and their ease of culture in standard commercially available clear nutrient broths, thereby facilitating the production of substantial quantities for use in the search for suitable binding components by inoculating the cultured amoeba with the target bacteria.

The bacteria binding components of the present invention have various advantages in relation to their use in the therapeutically active and other forms provided by the present invention, including substantial resistance to proteolytic enzymes and favourable binding characteristics. Thus in connection with the latter, where the SA bacterium binding component peptides of the invention have bound thereto Fc antibody fragment ligands, the bacterium binding component peptides moieties have been found to bind to a significant extent to the SA, despite competitive binding between the SA and the Fc moiety.

The bacteria binding components of the present invention (including both recombinant ones and ones recovered or isolated from bacteria predators), will generally be attached to another moiety or ligand for use in combating bacteria directly, or indirectly in any way, for example, by inclusion in diagnostic systems.

In a further aspect the present invention provides a method of diagnosing the presence of a target bacterium comprising the steps of:
providing a bacterium binding component peptide capable of specifically binding to said target bacterium;
bringing a said bacterium into contact with a said bacterium binding component peptide;
collecting bacterium binding component peptides bound to a said bacterium in a bacterium bound-bacterium binding component complex; and
detecting said bacterium bound-bacterium binding component complex.

It will be appreciated that there are various different types of diagnostic system which may be used for the detection of a target moiety in a sample. On the one hand the sample bacterium may be anchored to a substrate and then contacted by a detector moiety which binds specifically to the target and has attached thereto a label which can be read. After removing unbound sample and detector material from the substrate, the presence of any label will indicate the presence in the sample of the target bacterium. On the other hand there may be used a diagnostic system in which the detector moiety is attached to a substrate and after contacting with the sample and removal of any unbound material, the system is monitored for the presence of any bacteria attached (indirectly) to the substrate, for example by using a suitable stain to visualize any bacteria present. The substrate may be a fixed substrate such as a plate, slide, strip, etc, or could be a particulate substrate adapted to facilitate recovery thereof from a sample medium etc, for example by being tagged or labelled with magnetic material so that the substrate (together with any material attached thereto) may be easily captured by bringing a magnetic device into proximity therewith. It would in principle also be possible to use other separation techniques based on changes in physical properties resulting from binding of target bacterium to free labelled (or unlabelled) bacterium binding components using well known techniques such as gel electrophoresis, chromatography, mass spectrometry, centrifugation, etc, although these are generally less convenient.

Thus in the case of diagnostic systems, the bacteria binding component peptide would generally have attached thereto a label which can be more or less readily detected.

Alternatively the bacterium may be detected where the complexes are collected without co-collection of uncomplexed bacterium by suitable more or less non-specific staining using stains such as methylene blue or flourescent stains such as DAPI (4,6-Diamidino-2-phenylindole).

A variety of labelling systems suitable for use in the method of the invention are well known in the art including radioactive labels such as P32 or P33, fluorescent labels such as fluorescein or rhodamine and coloured dye label systems such as biotinylation followed by development with Streptavdin-Horseradish peroxidase conjugate and peroxidase substrate.

Thus in some cases, where, for example, a radioactive label is used, the presence of bound label may be detected with suitable apparatus such as a scintillation counter. In other cases there may require to be used a developer reagent system for "developing" the bound label into a form which can be detected by simple visual inspection and/or with the aid of suitable apparatus e.g. spectroscopic apparatus.

It will be appreciated that such a diagnostic method will be particularly useful in diagnosing bacterial infections of humans, animals and plants. The bacteria being probed for in human and animal infections would generally be found in carriers such as body fluids such as blood, lymph, urine, secretions, etc, or tissue samples suitably prepared in fluid or solid form such as tissue sections.

The diagnostic method of the present invention may also conveniently be used prior to therapeutic treatment incorporating bacterium binding component peptide. By pre-diagnosing an infection, the unnecessary administration of a therapeutic form of the bacterium binding component peptide to a patient uninfected with a particular bacterium may be avoided.

Diagnostic methods of the invention may also be used to diagnose contamination of water, food and drink products, industrial fluids, ventilation systems, etc.

In the case of active wound dressings, the bacterium binding component is used in an immobilised form bound to a wound contacting portion of the fabric of the dressing, generally by means of covalent coupling either through sulphyrdyl derivation or the use of iodoacetamide linkage so that bacteria present in the wound become bound to the dressing, and then removed from the wound upon changing of the wound dressing.

For therapeutic purposes the bacterium binding component would generally be used in a therapeutically active form comprising a bacterium binding component of the invention having an active moiety bound thereto, wherein the active moiety is a substance which can directly or indirectly lead to inactivation of the target bacterium. Thus in one preferred form of the invention, the bacteria binding component may have conjugated to it an Fc antibody fragment so that when the bacterium binding component peptide moiety becomes attached to a bacterium, it may then be recognised and duly phagocytosed by the body's white blood cells. Such a chineric protein may also activate the complement cascade providing a further route to inactivate the target bacterium. Conveniently where the bacterium binding component is a peptide this is conjugated to an Fc antibody fragment by means of splicing a predator gene fragment encoding the bacterium binding component peptide, onto a human gene fragment encoding the Fc antibody fragment and introducing the chimeric gene into an expression vector, and then recovering the expressed chimeric peptide product.

Advantageously, in order to reduce the affinity of the bacterium for the Fc antibody fragment, the latter is preferably employed in a variant form with selectively reduced affinity towards said bacterium, but in which its immmological activity (affinity for white blood cells) is substantially retained. Suitable varients may be obtained by means of site specific mutagenesis (see for example P. Carter et al (1985) "Improved oligonucleotide site-directed mutagenesis using M13 vectors." Nucleic Acids Res 13, 4431-4443).

Alternatively the bacterium binding component could be conjugated to a physiologically acceptable bacteriolytic moiety.

One suitable lysozyme-like (lytic) protein suitable for use as a physiologically acceptable toxin in accordance with the present invention is that obtainable from certain species of amoeba as reported in the literature Drozanski, W (1969). "Bacteriolytic enzyme produced by Acanthamoeba castellanii." Acta. Microbiolgica Polonica SerA 1(18), 155-168. We have also found a similar lysozyme-like protein effective in lysing S. aureus bacteria, in the Ven strain of Acanthamoeba from which we have recovered the BBP having the amino-acid sequence described herein.

Bacteria binding components of the present invention may also be used to provide antibiotic agents by conjugating them with physiologically acceptable toxins and other more toxic anti-bacterial toxins, in cleaning materials including cleaning fluids and anti-microbial cleaning cloths and the like, for general use, and especially for use in high risk areas such as hospitals and other medical facilities.

Many methods of chemical conjugation are well known in the art e.g. by means of covalent coupling either through sulphydryl derivation or the use of iodoacetamide linkage.

It is also possible to produce such antibacterial agents by recombinant techniques such recombinant agents have the advantage over conjugates produced by chemical procedures in that they are more readily produced than the conjugates, and homogeneous populations of the agent molecules can be produced.

The therapeutic products of the present invention may be administered to'the patient in any suitable form. The various therapeutic agents of the invention can be administered using conventional modes of administration, including but not limited to, intravenous, intraperitoneal, oral, intralymphatic or administration directly into the site of disease. Intravenous administration is generally preferred for use in systemic infections such as septicaemia as well as more localised infections, and topical formulations may be used in the treatment of wounds. In general topical formulations utilize an inert carrier such as petroleum gel or lanolin.

The compositions of the invention can be in a variety of dosage forms which include, but are not limited to, liquid solutions or suspensions, tablets, pills, powders, suppositories, polymeric microcapsules or microvesicles, liposomes and injectable or infusible solutions. The preferred form depends upon the mode of administration and the therapeutic application.

Such compositions can also include various buffers, excipients, additives, preservatives and other substances in accordance with normal practice in order to stabilize the pharmaceutical composition etc.

The most effective mode of administration and dosage regimen for the compositions of this invention depends upon the severity and course of the disease, the patient's health and response to treatment and the judgment of the treating physician.

In general the compositions of the invention comprise a bacterium binding component of the present invention in intimate admixture with a physiologically acceptable carrier therefor.

SA is also a problem in animal husbandry giving rise to, for example, conditions such as mastitis and accordingly the present invention also encompasses veterinary formulations comprising therapeutically active forms of bacterium binding components of the present invention, in intimate admixture with a physiologically acceptable carrier therefor.

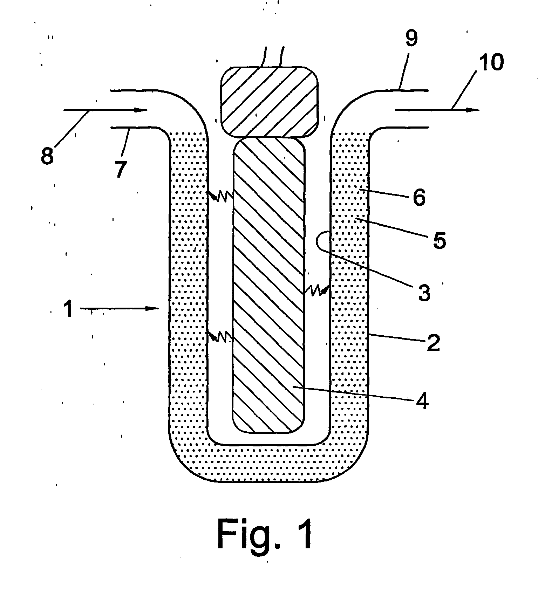

Another application of the bacterium binding components of the present invention which may be mentioned is in connection with water purification systems. In this case the bacterium binding component peptide is used in an immobilised form bound onto a substantially inert substrate, such as silica beads, by any suitable means, such as through covalent coupling either by sulphyrdyl derivation or the use of iodoacetamide linkage, which is supported in an irradiation zone, conveniently an UV irradiation zone, inside a water flow passage, as illustrated schematically in Fig. 1 which shows water sterilisation apparatus 1 comprising a conduit 2 having a wall 3 of UV-radiation transmitting material, and an UV radiation source 4 in close proximity to the wall. The conduit is filled with silica beads 5 constituting a substrate on which is immobilised a bacterium binding component peptide 6 such as that provided by Example 2 hereinbelow. The conduit 2 has an inlet 7 for receiving a flow of water 8 requiring treatment, and an outlet 9 for delivering treated water 10.

Bacteria present in the water are bound to a greater or lesser degree to the immobilised bacterium binding component peptides thereby substantially increasing their residence time within the irradiation zone whereby the efficiency of the bacterial inactivation treatment is significantly increased e.g. by allowing greater flow rates to be used. Various UV irradiation systems for treating water and other fluids, are well known in the art and commercially available.

Thus in a further aspect the present invention also provides a water sterilisation apparatus comprising:
a conduit having a wall of UV-radiation transmitting material; an external UV radiation source opposite said wall; and a substrate mounted within said conduit apposite said wall, said substrate having immobilised thereon a peptide having the following sequence: 
GSTGVHLDDVVIGSFQASPRQVSVSLSCFGDSGKPSGPMVHHVAGSELMAFSRIAFESASSQ
SHYLGAGFQRLRASGACPWGHGAWPCGPYLHPEGHCPGQVQHRMPVKAGVRLVDCPGRTGVV
VGHRVPQVCPVQSIIGIAVPRTGRRHVVREWTMNIA
 and said substrate being formed and arranged for providing an extended water-bacterium binding component peptide interface for passage of water flowing through said conduit thereover.

It will be appreciated that various forms of substrate suitable for maximising contact of a water flow therewith, whilst maintaining a reasonable flow of water therethrough, are well known in the art. Thus for example there may be used silica beads, such as those available from "The Sigma Chemical Company" or "Pharmacia", (Uppsala, Sweden).

If desired, the UV stability of the bacterium binding peptides used in the water sterilisation apparatus of the invention may be improved by substituting at least some amino acids containing aromatic moieties (such as tryptopham, tyrosine, and phenylalanine) with other amino acids, where possible without substantially reducing the binding capacity of the peptide for the target bacterium. It will also be understood that different UV radiation sources (such as UVA, UVB and UVC lamps) may be used as appropriate to reduce damage to more susceptible BBPs, albeit at the cost of some reduced bacterium inactivation effect.

In a preferred aspect the present invention provides a bacterium binding component peptide with the amino acid sequence 
GSTGVHLDDVVIGSFQASPRQVSVSLSCFGDSGKPSGPMVHHVAGSELMAFSRIAFESASSQ
SHYLGAGFQRLRASGACPWGHGAWPCGPYLHPEGHCPGQVQHRMPVKAGVRLVDCPGRTGVV
VGHRVPQVCPVQSIIGIAVPRTGRRHVVREWTMNIA
 which binds onto Staphylococcus aureus (SA) as well as therapeutically active forms thereof, and pharmaceutical formulations containing said therapeutically active forms. Also provided are diagnostic assays containing the SA bacterium binding component peptide bound to a label, and immobilised forms of the bacterium binding component peptide suitable for use in wound dressings and water purification systems, etc.

In a further preferred aspect of the invention the SA bacteria binding component peptide is a peptide having the amino acid sequence set forth hereinbefore. In another preferred aspect of the invention there is provided a cDNA having the nucleic acid sequence set forth hereinbefore.

The invention also provides a chimeric peptide comprising a SA bacterium binding component peptide having the amino acid set forth hereinbefore, bonded at its C terminal to an Fc peptide, preferably a human Fc peptide.

Further preferred features and advantages of the invention will appear from the following detailed examples given by way of illustration.

### Example 1 - Identification of Amoebae with BBPs for Target Bacteria

### A. Preparation of Amoeba Library

A library of (mainly) Acanthamoeba strains was established from a large number of soil samples as follows.

Soil samples (ca. 0.05g) were taken up in 10ml of saline solution and this deposited on agar plates containing 0.01% maltose and 0.01% yeast extract. Plates were incubated for 2-3 days to permit the amoeba to multiply on the accompanying bacteria from the soil sample.

Blocks (ca. 1cm²) were cut from the agar plate surface and this was inverted onto a fresh agar plate onto which a layer of E. coli (YM109) had been placed. (E-coli provides nutrition for the vast majority of phagocytic micro-organisms including Acanthamoeba).

Amobea "grew out" from under the block and crawled onto the surface of the agar plate, multiplying as they did so. These could be observed under the microscope. Amoebae were classified by their appearance (Acanthamoeba are visually characteristic as are Naegleria). Blocks of agar on which amoebae were observed were then inverted onto sterile plates containing a layer of heat killed E. coli. Once again the amoebae crawled and grew out from the inverted agar block leaving the living E. coli behind. Blocks of the sterile agar blocks containing the amoebae were then placed in axenic media to establish a culture in which the amoebae were the only living organism. Cells were cloned after a thriving culture was achieved by dilution.

### B. Screening the Amoebae Library

Agar plates onto which a layer of Staphlococcus or Enterococcus had been deposited were inoculated by placing a 100µl drop of each amoeba culture in the centre. After a period of from 2 days to 2 weeks, depending on the amoeba and bacteria, growth of the amoeba was seen as a clearing area on the plates. Amoeba that grew fastest on these plates were selected for isolation of BBPs.

### Example 2 - Isolation of Staphylococcus Aureus Binding Peptide

2.5 litre cultures of the Ven strain of *Acanthamoeba* (obtained from a soil sample from the side of a Venezuelan mountain) were grown in medium (peptone, yeast extract and glucose), collected by centrifugation, and homogenised in a buffer containing protease inhibitors. Large fragments including the nuclei were removed by centrifugation. The extract was then supplemented with washed, S.aureus bacteria that had been fixed in formaldehyde (by the method of Kessler, S.W. (1981) Methods Enzymology 73, 442-458). The bacteria and any attached bacterium binding component protein or peptide (BBP) from the amoebae were then pelleted out by centrifugation. The supernatant was discarded and the bacterial pellets washed in buffer. Proteins from the amoebae now specifically bound to the used amoebae before bacteria were stripped off in a buffer that contained 1% Na Deoxycholate, 0.1% SDS and 1% Triton X-100 detergents, (no bacterial proteins were present despite these harsh conditions because of the use of fixed bacteria). 12% SDS-Page gels (Laemmli, (1970) Nature 227, 680-685) were then run in order to assay the purity of the BBP, and these were further purified according to their molecular size by size exclusion chromatography (GEL filtration, using S-300, Pharmacia, Uppsala, Sweden). The BBP was then concentrated using centricon 10 (Amicon,Beverly, MA, US) filter units.

### Example 3 - Sequencing of Staphylococcus aureus Binding Peptide

Peptide fragments were generated from the pure BBP obtained in Example 2 by cyanogen bromide digestion, after which the peptides were separated by SDS-Page and blotted onto filters. The peptides were then stained with Coomassie blue dye and the peptide containing bands excised for sequencing by a commercial operator.

Two amino acid sequences were obtained with the following sequences.
PQLGDNVEKA
and
DRSWGWSPSN

### Example 4 - Cloning of Staphylococcus aureus Binding Peptide

### A. Construction of cDNA libraries from the Ven strain of Acanthamoeba

Amoebae were cultured as above and the total RNA isolated from the cells by "Stratagene Poly(A)quik kit" (La Jolla, CA). The mRNA was separated from the total RNA by polyT affinity chromatograpy (using a Poly(A)Quik kit, Stratagene, CA, US). The mRNA template was then used to produce cDNAs by reverse transcriptase (Gibco, Scotland). The cDNAs were then ligated with EcoR1 arms, and then ligated en masse into Bluescript (Stratagene, CA, US) vector.

### B. Screening of cDNA Library

The cDNA Library was screened by designing oligonucleotide primers based on the two short amino-acid sequences produced by Edman degradative sequencing of the BBP. The oligonucleotides used had the following nucleotide sequences.
CCCCAGCT (C/G) GG (C/G) GACAACGT (C/G) GAGAAGGC (C/G)
and
GACCG (C/G) TC (C/G) TGGGG (C/G) TGGTC (C/G) CCCTC (C/G)AAC

PCR was also used to confirm relationships between the cDNA fragments by standard procedures.

### C. Expression of BBP cDNAs in E.coli.

A fragment of the cDNA (judged to constitute a single domain on the basis of homology with other proteins) was expressed in *E.coli* (BL21-de3) using the T7 based vector, pMW172 (Way et al, EMBOJ 9, 4103-4109). The resulting protein was found to bind *S.* aureus by using the method described to isolate the Staphylococcus aureus binding proteins (see below).

### Example 5 - Binding of Staphylococcus aureus Binding Peptide to Bacteria

This was carried out essentially as for the SA binding protein identification as described in Example 2 except that instead of adding an amoebal lysate to the fixed bacteria, a lysate of bacteria expressing the putative binding proteins from Example 4, was added. BBP was then eluted off the fixed bacteria using the same buffers as in Example 2 and the result analysed by SDS-PAGE which yielded a clearly visible band indicating strong binding of the SA BBP to the bacteria.

### Example 6 - Preparation of Antibody to Staphylococcus aureus Binding Peptide

The BBP was purified from lysates of bacteria used to express the protein from Example 4. The purified and concentrated BBP was sent to a commercial operator who made chicken antibodies through their eggs. Antibody was recovered from the hen eggs and purified for use in binding affinity tests to demonstrate the immunogenic identity of the originally isolated native BBP, had been preserved.

### Example 7 - Preparation of Staphylococcus aureus "Antibody" Suitable for Therapeutic Use

The cDNA encoding the SA binding protein was ligated to a cDNA encoding the Fc region of a human antibody so that a continuous protein was produced, the N-terminus of which was SA binding protein and the C-terminal portion was Fc region. This construct was then ligated into the pMW172 vector (Way et al, EMBOJ 9, 4103-4109)that had been modified to place the OMPA secretion encoding signal onto the N-terminus of the chimeric protein. The signal caused the nascent amino-acid chain to be transported through the inner bacterial membrane into the periplamsmic space where conditions are compatible with the disulphide bonds necessary for the folding of the Fc region (Ghrayeb et al,1984 EMBOJ 3, 2437-2442). The chimeric protein construct was transformed into E.coli (JM109 de3) and grown in TB media. The soluble protein from the periplasm was purified by standard methods (Neu & Heppel, 1965 J.Biol.Chem. 240, 3685-3692).

### Example 8 - Preparation of Staphylococcus aureus "Antibody" Suitable for Therapeutic Use

As an alternative to the procedure of Example 7, the chimeric protein construct was ligated into the pPIK9 vector which was transformed into Pichia pastoris and integrated into the yeast genome. Transformed Pichia pastoris were grown in BMGY medium (commercially available) and integration was screened for by PCR using Ven amoeba cDNA specific primers. When the yeast carbon source was switched to methanol (BMMY medium commercially available) the chimeric protein product was secreted into the media and colonies of yeast selected on the basis of their ability to produce the protein.

The protein was purified from the culture media by precipitation with ammonium sulphate (50% w/v), extensive dialysis, chromatography using DE52, (Whatman) and gel filtration using S-200 (Pharmacia). The product was then lyophilised to dryness. The products was shown to have full activity after rehydration in physiological saline.

### Example 9 - Isolation of Pseudomonas fluorescence Binding Peptide

The procedure of Example 2 was followed using Pseudomonas fluorescence_bacteria to probe the homogenised Acanthamoebal preparation in place of the S. aureus bacteria, and a purified Pseudomonas fluorescence_BBP obtained.

### Example 10 - Preparation of Parenteral Formulation

The product obtained in Example 8 was dissolved in physiological saline (10 mg/ml) and packaged into injection vials.

## Claims

1. A method of recovering a bacterium binding component suitable for use in the preparation of a product for use in combating a target bacterium, from an eukaryotic micro-organism, which method comprises the steps of: providing a predator eukaryotic micro-organism candidate
for specific binding of said target bacterium; contacting bacterium binding components of said predator eukaryotic micro-organism with cell surface components of said target bacterium for specific binding of said target bacterium surface components with at least one said bacterium binding component of said at least one predator eukaryotic micro-organism so as to form a complex;
lysing said at least one eukaryotic micro-organism to form a lysate of said eukaryotic micro-organism; separating the complex from the remainder of said lysate; treating the complex so as to release said at least one bacterium binding component of said eukaryotic micro-organism from said bacterium surface components; and recovering said at least one bacterium binding component of said eukaryotic micro-organism.

2. A method according to claim 1 which includes the preliminary step of recovering at least one eukaryotic micro-organism from a habitat of said bacterium.

3. A method according to claim 1 or claim 2 which includes the preliminary step of screening a plurality of eukaryotic micro-organisms for at least one eukaryotic micro-organism which specifically binds said target bacterium.

4. A method according to claim 3 wherein said screening step comprises culturing said target bacterium in contact with said plurality of eukaryotic micro-organisms in the substantial absence of nutrient medium and selecting eukaryotic micro-organisms which are predatory on said bacteria and can thrive by means of predation on said target bacteria.

5. A method according to any one of claims 1 to 4 wherein said eukaryotic micro-organism is lysed before contacting of the bacterium binding components thereof with said target bacterium.

6. A method according to any one of claims 1 to 5 wherein said cell surface components of said target bacterium are used in a form in which they are part of the target bacterium cells.

7. A method of recovering a bacterium binding component suitable for use in the preparation of a product for use in combating the bacterium including the inactivation of said bacterium and/or the treatment or diagnosis of an infection by said bacterium, from an eukaryotic micro-organism, which method comprises the steps of:
recovering from a habitat of said bacterium a predator eukaryotic micro-organism that is predatory on a target bacterium and can thrive by means of predation on said target bacterium, wherein the predator eukaryotic micro-organism has at least one bacterium-binding component that specifically binds said target bacterium;
lysing said predator eukaryotic micro-organism to form a lysate containing said at least one bacterium-binding component;
contacting the lysate with said target bacterium for specific binding thereto of at least one bacterium binding component of said predator micro-organism to form a complex together therewith;
separating the complex from the remainder of said lysate; treating the separated complex so as to release said at least one bacterium binding component from said bacterium; and
recovering said at least one bacterium binding component.

8. A method according to any one of claims 1 to 7 wherein the recovered bacterium binding component is further broken down and screened again against said bacterium for identification of at least one active bacterium binding component region which specifically binds said target bacterium.

9. A method according to any one of claims 1 to 8 wherein the bacterium binding component comprises a peptide which peptide consists essentially of a peptide chain having a length not less than that required to provide a stable folding unit under normal physiological conditions.

10. A method according to any one of claims 1 to 9 which includes the further steps of: sequencing a recovered bacterium binding component peptide, so as to obtain the amino acid sequence thereof;
obtaining DNA coding for said bacteria binding component amino acid sequence;
introducing said DNA into an expression vector; and recovering recombinant bacteria binding component peptide produced by said expression vector.

11. A method according to any one of claims 1 to 10 wherein the eukaryotic micro-organism is selected from amoeba and protozoa species.

12. A method according to claim 11 wherein the eukaryotic micro-organism is an amoeba selected from Acanthamoeba and Naegleria genera.

13. A peptide for use in binding to Staphylococcus aureus and having an active bacterial binding domain with the amino acid sequence

GSTGVHLDDVVIGSFQASPRQVSVSLSCFGDSGKPSGPMVHHVAGSELMAFSRIAFE
SASSQSHYLGAGFQRLRASGACPWGHGAWPCGPYLHPEGHCPGQVQHRMPVKAGVRL
VDCPGRTGVVVGHRVPQVCPVQSIIGIAVPRTGRRHVVREWTMNIA.

14. The peptide according to claim 13 for use in a diagnostic system, wherein said peptide has a label attached thereto.

15. The peptide according to claim 13 for use in a diagnostic system, wherein said peptide is attached to a substrate selected from a fixed substrate and a particulate substrate formed and arranged so as to facilitate recovery thereof from a liquid medium.

16. The peptide according to claim 13 for use in the treatment or prophylaxis of a bacterial infection in an infected subject, wherein said peptide has a recognition element conjugated, directly or indirectly, thereto, which recognition element is recognizable by the subject's immune system for activation thereof.

17. The peptide according to claim 16 wherein said recognition element comprises an Fc antibody fragment.

18. The peptide according to claim 13 for use in the treatment or prophylaxis of a bacterial infection in an infected subject, wherein said peptide has a physiologically acceptable anti-bacterial toxin conjugated, directly or indirectly, thereto.

19. A pharmaceutical composition comprising a peptide according to any one of claims 16 - 18, in intimate admixture with a physiologically acceptable carrier therefor.

20. A water sterilisation apparatus comprising:
a conduit having a wall of UV-radiation transmitting material;
an external UV radiation source opposite said wall; and a substrate mounted within said conduit opposite said wall, said substrate having immobilised thereon a peptide of claim 13, and said substrate being formed and arranged for providing an extended water-bacterium binding component interface for passage of water flowing through said conduit thereover.

21. A cDNA sequence encoding the active bacterium binding domain of a bacterium binding component peptide, which cDNA has the nucleotide sequence:

GGCTCCACGGGAGTCCATCTGGACGACGTCGTCATCGGCAGTTTCCAGGCCAGCCCT
CGTCAGGTAAGTGTTAGCCTGAGTTGCTTTGGAGACTCAGGAAAACCTAGTGGCCC
ATGGTGCACCATGTTGCAGGCTCAGAGTTGATGGCCTTCTCCCGGATCGCGTTCGAA
TCAGCCTCGAGCCAGTCGCACTACCTGGGTGCAGGATTCCAGAGGTTGAGAGCTTCC

GGAGCTTGCCCTTGGGGGCATGGTGCTTGGCCCTGTGGTCCCTACCTGCACCCAGAG
GGCCATTGCCCGGGACAGGTCCAGCATCGGATGCCCGTCAAGGCGGGTGTCAGACTC
GTCGACTGCCCGGGCCGGACTGGCGTCGTTGTGGGCCATCGGGTTCCACAGGTGTGT
CCGGTTCAGTCAATCATAGGCATTGCTGTTCCAAGGACAGGACGCCGCCATGTTGTT
CGGGAGTGGACCATGAACATCGCC

22. A method according to any one of Claims 1 to 11, which includes the preliminary step of fixing said target bacterium prior to contacting with said eukaryotic micro-organism, so as make said bacterium resistant to lysis during lysis of the eukaryotic micro-organism, and to make said bacterium resistant to lysis during said treatment of said complex so as to release said bacterium binding component of said eukaryotic micro-organism from said bacterium cell surface components.

23. A method according to any one of Claims 1 to 11, or 22, which includes the step of treating the complex so as to differentially release any material which may only be bound non-specifically to the target bacterium, prior to said treatment of said complex so as to release said specifically bound bacterium binding component of said eukaryotic micro-organism from said bacterium surface components.

## Patentansprüche

1. Verfahren zum Gewinnen einer Bakterienbindungskomponente, geeignet zur Verwendung bei der Herstellung eines Produkts zur Venvendung beim Bekämpfen eines Zielbakteriums, aus einem eukaryotischen Mikroorganismus, welches Verfahren die Schritte umfaßt:
Bereitstellen eines Kandidaten für einen eukaryotischen Räubermikroorganismus zum spezifischen Binden des Zielbakteriums;
Inkontaktbringen von Bakterienbindungskomponenten des eukaryotischen Räubermikroorganismus mit Zelloberflächenkomponenten des Zielbakteriums zum spezifischen Binden von Oberflächenkomponenten des Zielbakteriums mit mindestens einer Bakterienbindungskomponente von dem mindestens einen eukaryotischen Räubermikroorganismus, um einen Komplex zu bilden;
Lysieren des mindestens einen eukaryotischen Mikroorganismus, um ein Lysat des eukaryotischen Mikroorganismus zu bilden;
Abtrennen des Komplexes von dem Rest des Lysats;
Behandeln des Komplexes, um die mindestens eine Bakterienbindungskomponente des eukaryotischen Mikroorganismus von den Bakterienoberflächenkomponenten freizusetzen; und
Gewinnen der mindestens einen Bakterienbindungskomponente des eukaryotischen Mikroorganismus.

2. Verfahren nach Anspruch 1, welches den vorbereitenden Schritt einschließt, mindestens einen eukaryotischen Mikroorganismus aus einem Habitat des Bakteriums zu gewinnen.

3. Verfahren nach Anspruch 1 oder Anspruch 2, welches den vorbereitenden Schritt einschließt, eine Mehrzahl von eukaryotischen Mikroorganismen für mindestens einen eukaryotischen Mikroorganismus zu screenen, welcher das Zielbakterium spezifisch bindet.

4. Verfahren nach Anspruch 3, wobei der Schritt des Screenens umfaßt, das Zielbakterium in Kontakt mit der Mehrzahl von eukaryotischen Mikroorganismen im wesentlichen in Abwesenheit von Nährmedium zu kultivieren und eukaryotische Mikroorganismen auszuwählen, welche auf den Bakterien räuberisch sind und mittels Räubertum auf den Zielbakterien gedeihen können.

5. Verfahren nach einem der Ansprüche 1 bis 4, wobei der eukaryotische Mikroorganismus lysiert wird, bevor die Bakterienbindungskomponenten davon mit dem Zielbakterium in Kontakt gebracht werden.

6. Verfahren nach einem der Ansprüche 1 bis 5, wobei die Zelloberflächenkomponenten des Zielbakteriums in einer Form verwendet werden, in welcher sie Teil der Zielbakterienzellen sind.

7. Verfahren zum Gewinnen einer Bakterienbindungskomponente, geeignet zur Verwendung bei der Herstellung eines Produkts zur Verwendung beim Bekämpfen des Bakteriums, einschließend die Inaktivierung des Bakteriums und/oder die Behandlung oder Diagnose einer Infektion durch das Bakterium, aus einem eukaryotischen Mikroorganismus, welches Verfahren die Schritte umfaßt:
Gewinnen aus einem Habitat des Bakteriums von einem eukaryotischen Räubermikroorganismus, der auf einem Zielbakterium räuberisch ist und mittels Räubertum auf dem Zietbakterium gedeihen kann, wobei der eukaryotische Räubermikroorganismus mindestens eine Bakterienbindungskomponente aufweist, die das Zielbakterium spezifisch bindet;
Lysieren des eukaryotischen Räubermikroorganismus, um ein Lysat, enthaltend die mindestens eine Bakterienbindungskomponente, zu bilden; Inkontaktbringen des Lysats mit dem Zielbakterium, um mindestens eine Bakterienbindungskomponente des Räubermikroorganismus spezifisch daran zu binden, um zusammen damit einen Komplex zu bilden;
Abtrennen des Komplexes von dem Rest des Lysats;
Behandeln des abgetrennten Komplexes, um die mindestens eine Bakterienbindungskomponente von dem Bakterium freizusetzen; und
Gewinnen der mindestens einen Bakterienbindungskomponente.

8. Verfahren nach einem der Ansprüche 1 bis 7, wobei die gewonnene Bakterienbindungskomponente weiter aufgespalten wird und wieder gegen das Bakterium gescreent wird, um mindestens eine aktive Region der Bakterienbindungskomponente, welche das Zielbakterium spezifisch bindet, zur identifizieren.

9. Verfahren nach einem der Ansprüche 1 bis 8, wobei die Bakterienbindungskomponente ein Peptid umfaßt, welches Peptid im wesentlichen aus einer Peptidkette besteht, die eine Länge aufweist, die nicht geringer als die ist, die erforderlich ist, um unter normalen physiologischen Bedingungen eine stabile Faltungseinheit bereitzustellen.

10. Verfahren nach einem der Ansprüche 1 bis 9, welches die weiteren Schritte einschließt:
Sequenzieren eines Peptids einer gewonnenen Bakterienbindungskomponente, um die Aminosäuresequenz davon zu erhalten;
Erhalten der DNA-Codierung für die Aminosäuresequenz der Bakterienbindungskomponente;
Einführen der DNA in einen Expressionsvektor; und
Gewinnen des Peptids der rekombinanten Bakterienbindungskomponente, erzeugt durch den Expressionsvektor.

11. Verfahren nach einem der Ansprüche 1 bis 10, wobei der eukaryotische Mikroorganismus aus Amöben- und Protozoa-Spezies ausgewählt ist.

12. Verfahren nach Anspruch 11, wobei der eukaryotische Mikroorganismus eine Amöbe, ausgewählt aus den Gattungen Acanthamoeba und Naegleria, ist.

13. Peptid zur Verwendung beim Binden an Staphylococcus aureus und aufweisend eine aktive bakterielle Bindungsdomäne mit der Antinosäuresequenz

GSTGVHLDDVVIGSFQASPRQVSVSLSCFGDSGKPSGPMVHHVAGSELMAFSRIA
FESASSQSHYLGAGFQRLRASGACPWGHGAWPCGPYLHPEGHCPGQVQHRMPV
KAGVRLVDCPGRTGVVVGHRVPQVCPVQSIIGIAVPRTGRRHVVREWTMNIA.

14. Peptid nach Anspruch 13 zur Verwendung in einem diagnostischen System, wobei das Peptid eine daran angefügte Markierung aufweist.

15. Peptid nach Anspruch 13 zur Verwendung in einem diagnostischen System, wobei das Peptid an ein Substrat, ausgewählt aus einem fixierten Substrat und einem teilchenförmigen Substrat, gebildet und angeordnet, um die Gewinnung davon aus einem flüssigen Medium zu erleichtern, angefügt ist.

16. Peptid nach Anspruch 13 zur Verwendung bei der Behandlung oder Prophylaxe einer bakteriellen Infektion bei einem infizierten Patienten, wobei das Peptid ein Erkennungselement, direkt oder indirekt damit konjugiert, aufweist, welches Erkennungselement durch das Immunsystem des Patienten zur Aktivierung davon erkennbar ist.

17. Peptid nach Anspruch 16, wobei das Erkennungselement ein Fc-Antikörperfragment umfaßt.

18. Peptid nach Anspruch 13 zur Verwendung bei der Behandlung oder Prophylaxe einer bakteriellen Infektion bei einem infizierten Patienten, wobei das Peptid ein physiologisch verträgliches antibakterielles Toxin, direkt oder indirekt damit konjugiert, aufweist.

19. Pharmazeutische Zusammensetzung, umfassend ein Peptid nach einem der Ansprüche 16 18 in innigem Gemisch mit einem physiologisch verträglichen Träger dafür.

20. Wassersterilisationsapparatur, umfassend:
eine Rohrleitung mit einer Wand aus für UV-Strahlung durchlässigem Material;
eine außen liegende UV-Strahlungsquelle gegenüber der Wand; und
ein Substrat, angebracht innerhalb der Rohrleitung gegenüber der Wand, wobei auf dem Substrat ein Peptid nach Anspruch 13 immobilisiert worden ist und wobei das Substrat gebildet und angeordnet ist, um eine ausgedehnte Wasser-Bakterienbindungskomponente-Grenzfläche zur Passage von Wasser, das durch die Rohrleitung darüberfließt, bereitzustellen,

21. cDNA-Sequenz, codierend die aktive Bakterienbindungsdomäne eines Peptids einer Bakterienbindungskomponente, welche cDNA die Nucleotidesequenz hat:

GGCTCCACGGGAGTCCATCTGGACGACGTCGTCATCGGCAGTTTCCAGGCCA
GCCCTCGTCAGGTAAGTGTTAGCCTGAGTTGCTTTGGAGACTCAGGAAAACCT

AGTGGGCCCATGGTGCACCATGTTGCAGGCTCAGAGTTGATGGCCTTCTCCCG
GATCGCGTTCGAATCAGCCTCGAGCCAGTCGCACTACCTGGGTGCAGGATTCC
AGAGGTTGAGAGCTTCCGGAGCTTGCCCTTGGGGGCATGGTGCTTGGCCCTGT
GGTCCCTACCTGCACCCAGAGGGCCATTGCCCGGGACAGGTCCAGCATCGGA
TGCCCGTCAAGGCGGGTGTCAGACTCGTCGACTGCCCGGGCCGGACTGGCGT
CGTTGTGGGCCATCGGGTTCCACAGGTGTGTCCGGTTCAGTCAATCATAGGCA
TTGCTGTTCCAAGGACAGGACGCCGCCATGTTGTTCGGGAGTGGACCATGAA
CATCGCC.

22. Verfahren nach einem der Ansprüche 1 bis 11, welches den vorbereitenden Schritt einschtießt, das Zietbakterium vor dem Inkontaktbringen mit dem eukaryotischen Mikroorganismus zu fixieren, um das Bakterium während der Lyse des eukaryotischen Mikroorganismus resistent gegen Lyse zu machen und das Bakterium während der Behandlung des Komplexes, um die Bakterienbindungskomponente des eukaryotischen Mikroorganismus von den Bakterienzelloberflächenkomponenten freizusetzen, resistent gegen Lyse zu machen.

23. Verfahren nach einem der Ansprüche 1 bis 11 oder 22, welches den Schritt einschließt, den Komplex zu behandeln, um alles Material, welches nur nicht-spezifisch an das Zielbakterium gebunden werden kann, differentiell freizusetzen, bevor der Komplex behandelt wird, um die spezifisch gebundene Bakterienbindungskomponente des eukaryotischen Mikroorganismus von den Bakterienoberflächenkomponenten freizusetzen.

## Revendications

1. Procédé de récupération d'un composant de liaison à une bactérie approprié à l'utilisation dans la préparation d'un produit pour l'utilisation dans le combat contre une bactérie cible, à partir d'un micro-organisme eucaryote, lequel procédé comprend les étapes de:
fourniture d'un candidat micro-organisme eucaryote prédateur pour la liaison spécifique de ladite bactérie cible;
mise en contact des composants de liaison à une bactérie dudit micro-organisme eucaryote prédateur avec les composants de la surface cellulaire de ladite bactérie cible pour la liaison spécifique desdits composants de la surface de la bactérie cible avec au moins un dudit composant de liaison à une bactérie dudit au moins un micro-organisme eucaryote prédateur afin de former un complexe;
lyse dudit au moins un micro-organisme eucaryote pour former un lysat dudit micro-organisme eucaryote;
séparation du complexe du reste dudit lysat;
traitement du complexe afin de libérer ledit au moins un composant de liaison à la bactérie dudit micro-organisme eucaryote desdits composants de la surface de la bactérie;
et
récupération dudit au moins un composant de liaison à la bactérie dudit micro-organisme eucaryote.

2. Procédé selon la revendication 1, qui inclut l'étape préliminaire de récupération d'au moins un micro-organisme eucaryote d'un habitat de ladite bactérie.

3. Procédé selon la revendication 1 ou la revendication 2, qui inclut l'étape préliminaire de criblage d'une pluralité de micro-organismes eucaryotes concernant au moins un micro-organisme eucaryote qui se lie de manière spécifique à ladite bactérie cible.

4. Procédé selon la revendication 3, dans lequel ladite étape de criblage comprend la culture de ladite bactérie cible en contact avec ladite pluralité de micro-organismes eucaryotes en l'absence substantielle de milieu nutritif et le choix de micro-organismes eucaryotes qui sont des prédateurs desdites bactéries et qui peuvent se développer par prédation à partir desdites bactéries cibles.

5. Procédé selon l'une quelconque des revendications 1 à 4, dans lequel ledit micro-organisme eucaryote est lysé avant la mise en contact des composants de celui-ci de liaison à une bactérie avec ladite bactérie cible.

6. Procédé selon l'une quelconque des revendications 1 à 5, dans lequel lesdits composants de la surface cellulaire de ladite bactérie cible sont utilisés en une forme dans laquelle ils constituent une partie des cellules de la bactérie cible.

7. Procédé de récupération d'un composant de liaison à une bactérie approprié à l'utilisation dans la préparation d'un produit pour l'utilisation dans le combat contre la bactérie incluant l'inactivation de ladite bactérie et/ou le traitement ou le diagnostic d'une infection par ladite bactérie, à partir d'un micro-organisme eucaryote, lequel procédé comprend les étapes de:
récupération à partir d'un habitat de ladite bactérie d'un micro-organisme eucaryote prédateur qui est prédateur de la bactérie cible et qui peut se développer par prédation à partir de ladite bactérie cible, dans lequel le micro-organisme eucaryote prédateur a au moins un composant de liaison à une bactérie qui se lie spécifiquement à ladite bactérie cible;
lyse dudit micro-organisme eucaryote prédateur afin de former un lysat contenant ledit au moins un composant de liaison à une bactérie;
mise en contact du lysat avec ladite bactérie cible pour la liaison spécifique à celle-ci d'au moins un composant de liaison à une bactérie dudit micro-organisme prédateur afin de former un complexe conjointement avec celle-ci;
séparation du complexe du reste dudit lysat;
traitement du complexe séparé afin de libérer ledit au moins un composant de liaison à une bactérie de ladite bactérie; et
récupération dudit au moins un composant de liaison à une bactérie.

8. Procédé selon l'une quelconque des revendications 1 à 7, dans lequel le composant récupéré de liaison à une bactérie est en outre brisé et à nouveau criblé concernant ladite bactérie pour l'identification d'au moins une région active de composant de liaison à une bactérie qui se lie spécifiquement à ladite bactérie cible.

9. Procédé selon l'une quelconque des revendications 1 à 8, dans lequel le composant de liaison à une bactérie comprend un peptide lequel peptide consiste essentiellement en une chaîne peptidique ayant une longueur non inférieure à celle requise pour fournir une unité de repliement stable des chaînes sous des conditions physiologiques normales.

10. Procédé selon l'une quelconque des revendications 1 à 9, qui inclut les étapes supplémentaires de:
séquençage d'un peptide récupéré composant de liaison à une bactérie, afin d'obtenir la séquence d'acides aminés de celui-ci;
obtention de l'ADN codant pour ladite séquence d'acides aminés de composant de liaison à des bactéries;
introduction dudit ADN dans un vecteur d'expression; et
récupération du peptide recombinant composant de liaison à des bactéries produit par ledit vecteur d'expression.

11. Procédé selon l'une quelconque des revendications 1 à 10, dans lequel le micro-organisme eucaryote est choisi parmi les espèces de type amibes et protozoaires.

12. Procédé selon la revendication 11, dans lequel le micro-organisme eucaryote est une amibe choisie parmi le genre *Acanthamoeba* et *Naegleria.*

13. Peptide pour l'utilisation dans la liaison au *Staphylococcus aureus* et ayant un domaine de liaison bactérien actif avec la séquence d'acides aminés

GSTGVHLDDVVIGSFQASPRQVSVSLSCFGDSGKPSGPMVHHVAGSELMAFSRIA
FESASSQSHYLGAGFQRLRASGACPWGHGAWPCGPYLHPEGHCPGQVQHRMPV
KAGVRLVDCPGRTGVVVGHRVPQVCPVQSIIGIAVPRTGRRHVVREWTMNIA.

14. Peptide selon la revendication 13, pour l'utilisation dans un système de diagnostic, dans lequel ledit peptide a un marqueur qui lui est fixé.

15. Peptide selon la revendication 13, pour l'utilisation dans un système de diagnostic, dans lequel ledit peptide est fixé à un substrat choisi parmi un substrat fixé et un substrat sous forme de particule formé et disposé de façon à faciliter la récupération de celui-ci à partir d'un milieu liquide.

16. Peptide selon la revendication 13, pour l'utilisation dans le traitement ou la prophylaxie d'une infection bactérienne chez un sujet infecté, dans lequel ledit peptide a un élément de reconnaissance conjugué, directement ou indirectement, à celui-ci, lequel élément de reconnaissance peut être reconnu par le système immunitaire du sujet pour l'activation de celui-ci.

17. Peptide selon la revendication 16, dans lequel ledit élément de reconnaissance comprend un fragment d'anticorps Fc.

18. Peptide selon la revendication 13, pour l'utilisation dans le traitement ou la prophylaxie d'une infection bactérienne chez un sujet infecté, dans lequel(laquelle) ledit peptide a une toxine antï-bactérïenne physiologiquement acceptable conjuguée, directement ou indirectement, à celui-ci.

19. Composition pharmaceutique comprenant un peptide selon l'une quelconque des revendications 16 à 18, en mélange par admixtion intime avec un support physiologiquement acceptable pour celui-ci,

20. Appareil de stérilisation d'eau comprenant:
un conduit ayant une paroi de matériau transmetteur de rayonnement UV;
une source de rayonnement UV externe opposée à ladite paroi; et
un substrat monté à l'intérieur dudit conduit opposé à ladite paroi, ledit substrat ayant immobilisé dessus un peptide selon la revendication 13, et ledit substrat étant formé et disposé afin de fournir une interface de composant de liaison eau-bactérie étendue pour le passage de l'eau s'écoulant à travers ledit conduit par dessus.

21. Séquence d'ADNc codant ledit domaine de liaison actif à une bactérie d'un peptide composant de liaison à la bactérie, lequel ADNc a la séquence nucléotidique:

GGCTCCACGGGAGTCCATCTGGACGACGTCGTCATCGGCAGTTTCCAGGCCA
GCCCTCGTCAGGTAAGTGTTAGCCTGAGTTGCTTTGGAGACTCAGGAAAACCT
AGTGGGCCCATGGTGCACCATGTTGCAGGCTCAGAGTTGATGGCCTTCTCCCG
GATCGCGTTCGAATCAGCCTCGAGCCAGTCGCACTACCTGGGTGCAGGATTCC
AGAGGTTGAGAGCTTCCGGAGCTTGCCCTTGGGGGCATGGTGCTTGGCCCTGT
GGTCCCTACCTGCACCCAGAGGGCCATTGCCCGGGACAGGTCCAGCATCGGA
TGCCCGTCAAGGCGGGTGTCAGACTCGTCGACTGCCCGGGCCGGACTGGCGT
CGTTGTGGGCCATCGGGTTCCACAGGTGTGTCCGGTTCAGTCAATCATAGGCA
TTGCTGTTCCAAGGACAGGACGCCGCCATGTTGTTCGGGAGTGGACCATGAA
CATCGCC.

22. Procédé selon l'une quelconque des revendications 1 à 11, qui inclut l'étape préliminaire de fixation de ladite bactérie cible avant la mise en contact avec ledit micro-organisme eucaryote, afin de rendre ladite bactérie résistante à la lyse durant la lyse du micro-organisme eucaryote, et pour rendre ladite bactérie résistante à la lyse durant ledit traitement dudit complexe afin de libérer ledit composant de liaison à la bactérie dudit micro-organisme eucaryote à partir desdits composants de la surface cellulaire de la bactérie.

23. Procédé selon l'une quelconque des revendications 1 à 11, ou 22, qui inclut l'étape de traitement du complexe afin de libérer de manière différentielle tout matériau qui pourrait uniquement être lié de manière non spécifique à la bactérie cible, avant ledit traitement dudit complexe afin de libérer ledit composant de liaison à la bactérie spécifiquement lié dudit micro-organisme eucaryote à partir desdits composants de la surface de la bactérie.
